(19) Europäisches Patentamt
European Patent Office
Office européen des brevets

(11) EP 2 581 375 A2

(12) # EUROPEAN PATENT APPLICATION

published in accordance with Art. 153(4) EPC

(43) Date of publication:
**17.04.2013 Bulletin 2013/16**

(21) Application number: **11791980.3**

(22) Date of filing: **13.06.2011**

(51) Int Cl.:
***C07D 471/08*** *(2006.01)*

(86) International application number:
**PCT/ES2011/070424**

(87) International publication number:
**WO 2011/154586 (15.12.2011 Gazette 2011/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2011 US 201113156739**
**14.06.2010 US 354442 P**
**11.06.2010 ES 201030911**

(71) Applicant: **Medichem, S.A.**
**08970 Sant Joan Despi, Barcelona (ES)**

(72) Inventors:
- **SOLDEVILLA MADRID, Nuria**
**E-08001 Barcelona (ES)**
- **VÁZQUEZ MARTÍNEZ, Manel**
**E-17180 Vilablareix (Gerona) (ES)**

(74) Representative: **Pons Ariño, Angel**
**Pons Patentes y Marcas Internacional, S.L.**
**Glorieta Rubén Dario 4**
**28010 Madrid (ES)**

(54) **IMPROVED METHODS FOR THE PREPARATION OF QUINOXALINE DERIVATIVES**

(57) The present invention provides an improved method for preparing a compound of formula (IIIA), which is a varenicline synthesis intermediate. Furthermore, the present invention provides an improved method for preparing varenicline, or a salt or a solvate thereof. Moreover, the present invention provides a process for decolouring varenicline, or a salt or a solvate thereof. Moreover, the present invention provides a process for preparing varenicline L-tartrate, with an improved yield. Moreover, the present invention relates to the use of a compound of formula (V), or a salt or a solvate thereof, as reference marker and reference standard for evaluating the purity of varenicline, or a salt or a solvate thereof.

EP 2 581 375 A2

## Description

The present invention relates to an improved process for preparing an intermediate of the synthesis of varenicline. Further, the present invention relates to an improved process for preparing varenicline, or a salt or a solvate thereof, to a process for decolorizing varenicline or a salt or solvate thereof, to a process for preparing varenicline L-tartrate with an improved yield, and to the use of a compound of formula (V), or a salt or solvate thereof, as a reference marker and reference standard for assessing the the purity of varenicline, or a salt or solvate thereof.

BACKGROUND OF THE INVENTION

Varenicline (a compound of formula (I) below) is the international commonly accepted non-proprietary name for 7,8,9,10-tetrahydro-6,10-methano-6*H*-pyrazino[2,3-*h*][3]benzazepine (which is also known as 5,8,14-triazatetracyclo [10.3.1.0$^{2,11}$.0$^{4,9}$]-hexadeca-2(11),3,5,7,9-pentaene), and has an empirical formula of $C_{13}H_{13}N_3$ and a molecular weight of 211.26.

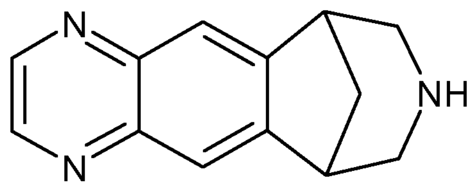

(I)

The L-tartrate salt of varenicline is known to be therapeutically useful and is commercially marketed for the treatment of smoking addiction. Varenicline L-tartrate is a partial agonist selective for $\alpha 4\beta 2$ nicotinic acetylcholine receptor subtypes. In the United States, varenicline L-tartrate is marketed under the name Chantix™, and is indicated as an aid to smoking cessation treatment.

Varenicline base and its pharmaceutically acceptable acid addition salts are described in U.S. Patent No. 6,410,550. In particular, Example 26 (Steps B and C) of this reference describes the preparation of varenicline by means of (i) cyclizing 10-(trifluoroacetyl)-10-azatricyclo[6.3.1.0$^{2,7}$]dodeca-2,4,6-triene-4,5-diamine (a compound of formula (IIa) as shown in Scheme 1 below) with glyoxal sodium bisulfite addition compound hydrate for 2.5 hours at 55°C in a monophasic mixture of tetrahydrofuran and water, (ii) isolating the obtained quinoxaline intermediate 8-(trifluoroacetyl)-7,8,9,10-tetrahydro-6*H*-6,10-methanoazepino[4,5-*g*]quinoxaline (a compound of formula (IIIa) as shown in Scheme 1 below) from the reaction mixture by extracting the monophasic aqueous mixture with ethyl acetate, washing the organic layer with water, drying with sodium sulphate, filtering and concentrating under vacuum, (iii) purifying a compound of formula (IIIa) by column chromatography and (iv) hydrolyzing a compound of formula (IIIa) with sodium carbonate in a mixture of methanol and water. The above synthetic route can be depicted in following Scheme 1.

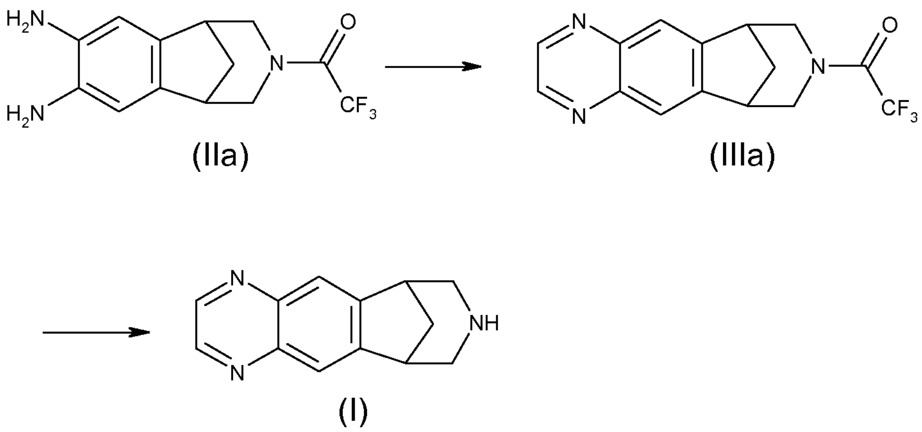

(IIa) (IIIa) (I)

Scheme 1

The same procedure as described in above Scheme 1 is also used in a number of further references for

preparing varenicline, such as U.S. Patent Nos. 7,091,372 and 7,186,870, and PCT Application Publication No. WO 2009/111623.

International Patent Application Publication No. WO 2006/090236 describes that during the known cyclization of a compound of formula (IIa) with aqueous glyoxal as depicted in Scheme 1 above, 7-(trifluoroacetyl)-1,5,6,7,8,9-hexahydro-5,9-methanoimidazo[4,5-*h*][3]benzazepine, which is a compound of formula (IVa)

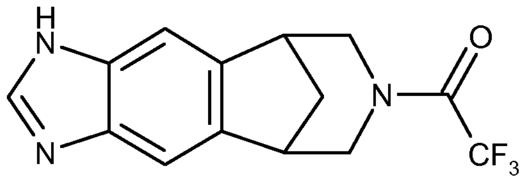

(IVa)

is formed as a by-product. WO 2006/090236 thus aims to provide an improved process for preparing varenicline, which provides varenicline with enhanced purity and that minimizes the formation of the above impurity (IVa). More specifically, WO 2006/090236 describes (i) cyclizing a compound of formula (IIa), or an analogue thereof having a nitrogen protecting group alternative to trifluoroacetyl, with aqueous glyoxal in a protic water miscible organic solvent, and (ii) removing the nitrogen protecting group by hydrolysis of the quinoxaline formed in step (i) with a base in a biphasic mixture of water and a water immiscible organic solvent. Furthermore, WO 2006/090236 describes that the reaction of step (i) not only needs to be carried out in the presence of aqueous glyoxal and in a protic water miscible organic solvent, but other technical features should be also controlled (i.e. the reaction should be conducted at a temperature range from -10°C to 20°C, the aqueous glyoxal should contain from 85% to 95% by weight water, and the pH should be maintained at a range of from 6 to 8). Precisely, Example 1 of WO 2006/090236 describes the cyclization of a compound of formula (IIa) by reacting a compound of formula (IIa) with aqueous glyoxal for 2 hours at 0-5°C and 18 hours at 20°C in a mixture of isopropyl alcohol and water, and isolating the obtained intermediate compound of formula (IIIa) by crystallization in a mixture of isopropyl alcohol and water which requires partially distilling the reaction solvent mixture, adding water to crystallize a compound of formula (IIIa), and filtering a compound of formula (IIIa) under vacuum. Furthermore, Example 4 describes the deprotection of a compound of formula (IIIa) by hydrolyzing a compound of formula (IIIa) with sodium hydroxide in a biphasic mixture of either toluene and water (step A) or dichloromethane and water (step B), to obtain varenicline, followed by decolorization of varenicline by means of treatment with active charcoal in methanol, and isolation by formation of its L-tartaric acid salt.

International Patent Application Publication No. WO 2009/155403 also describes an improved preparation of varenicline based on the process described in above Scheme 1 which includes an additional step of purifying a compound of formula (IIIa) by combining or contacting a compound of formula (IIIa) with an acid, such as hydrochloric acid, hydrobromic acid, hydroiodic acid, and sulfuric acid. In particular, Examples 14 and 15 of WO 2009/155403 carry out the purification of a compound of formula (IIIa) by means of extracting the reaction mixture with ethyl acetate, and washing with an aqueous solution of hydrochloric acid.

However, the processes described in the prior art for preparing a compound of formula (IIIa), or an analogue thereof, and varenicline of formula (I), from a compound of formula (IIa), or an analogue thereof, via the synthetic route of Scheme 1, present a number of drawbacks, which make them inefficient, low cost-effective and non suitable for industrial implementation. For example, in order to obtain a compound of formula (IIIa) with acceptable yield and purity and with a minimum presence of impurity (IVa), the processes of the prior art require the use of special cyclization conditions (for example, a protic, aqueous miscible solvent, the control of temperature from -10 to 20°C and also careful control of the pH). Furthermore, the prior art processes employ inefficient and costly purification steps, for example, column chromatography, crystallization and filtration which in turn require partial distillation of the employed solvent mixture followed by addition of an anti-solvent to facilitate precipitation and extraction of the product and also washing with aqueous acid solutions. Additionally, the processes described in the prior art for preparing varenicline via the synthetic route of above Scheme 1 require the manipulation of the solid quinoxoline intermediate of formula (IIIa), or an analogue thereof, since the cyclization processes are carried out in monophasic solvent mixtures which are not suitable for the subsequent hydrolysis in order to prepare varenicline, and consequently require the additional steps of isolating the intermediate compound of formula (IIIa), or an analogue thereof, and solvent replacement in order to carry out the final hydrolysis step. Also, the isolation of an intermediate of formula (IIIa), or an analogue thereof, requires partial distillation of the reaction solvent mixtures, which generates uneconomical solvent mixture residues.

In view of the above discussed state of the art relating to processes of preparing varenicline, there is, therefore, a need for providing improved processes for preparing an intermediate compound of formula (IIIa), and varenicline, from a compound of formula (IIa), or an analogue thereof, which could yield intermediate compound of formula (IIIa) and

varenicline with enhanced purity and yield and which could avoid the drawbacks of the prior art. Such processes to varenicline could thus facilitate cost-effective preparation of varenicline and as such could be suitable for industrial scale up.

Additionally, it is generally known that varenicline L-tartrate is usually obtained as a yellow solid. In this regard, colour is attributed to the presence of some specific impurities that may, or may not, be detectable by conventional methods such as HPLC. The presence of impurities may adversely affect the safety and shelf life of formulations. In this connection, and in order to remove coloured impurities from the varenicline L-tartrate, the majority of the processes described in the prior art introduce a step of decolorization in the synthesis of the varenicline L-tartrate. For example, International Patent Application Publication Nos. WO 2004/108725 and WO 2006/090236 describe the removal of colored impurities from varenicline by treating a methanolic solution of varenicline with activated carbon. Furthermore, International Patent Application Publication No. WO 2010/005643 describes the decoloration of a solution of varenicline, or its L-tartrate salt, in water, ethanol-water, methanol or mixtures thereof, with activated carbon. Based on such processes known in the art, there is also a need for an improved process for decolorizing varenicline, or its pharmaceutically acceptable salts.

## SUMMARY OF THE INVENTION

The present invention provides an improved process for preparing a compound of formula (IIIA), an intermediate of the synthesis of varenicline. Also, the present invention provides an improved process for preparing varenicline, or a pharmaceutically acceptable salt or solvate thereof. Furthermore, the present invention provides a process for decolorizing varenicline, or a salt or solvate thereof. Still further, the present invention provides a process of preparing varenicline L-tartrate with improved yield. Still further, the present invention relates to the use of compound of formula (V), or a salt or solvate thereof, as a reference marker and reference standard for assessing the purity of varenicline, or a salt or solvate thereof.

## DETAILED DESCRIPTION OF THE INVENTION

According to the present invention, there is, therefore, provided a process for preparing a compound of formula (IIIA)

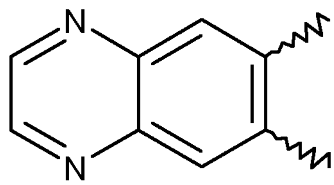

(IIIA)

the process comprising cyclizing a compound of formula (IIA)

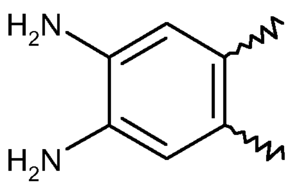

(IIA)

with a cyclizing agent, so as to obtain a compound of formula (IIIA),
characterized in that the cyclization is carried out in the presence of a biphasic solvent system comprising an aqueous phase and an organic phase.

Further details of the family of compound suitable for the present process are disclosed in the US 20070185327, which is herein incorporate by reference.

A process according to the present invention preferably comprises a process wherein said compound of formula (IIIA) is a compound of formula (III)

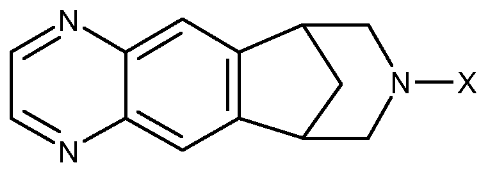

(III)

and said compound of formula (IIA) is a compound of formula (II)

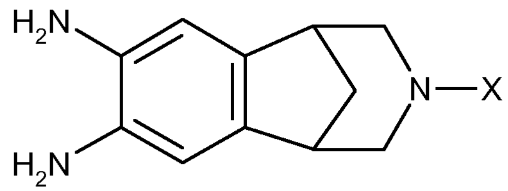

(II)

wherein X is an amino protecting group.

A process of preparing a compound of formula (III) according to the present invention preferably further comprises treating a compound of formula (III) with an amino deprotecting agent suitable for removing the amino protecting group of the compound of formula (III), preferably by hydrolysis, so as to yield varenicline, or a salt or solvate thereof, which deprotection is optionally carried out in the presence of a biphasic solvent system comprising an aqueous phase and an organic phase. It is further preferred that the deprotection is carried out in the same biphasic solvent system as present for the cyclization of a compound of formula (II) to yield a compound of formula (III) substantially as hereinbefore described, whereby varenicline, or a salt or solvate thereof, is prepared in a one-pot process from a compound of formula (II) via a compound of formula (III).

According to the present invention, there is, therefore, further provided a process of preparing varenicline, or a salt or solvate thereof, which process comprises cyclizing a compound of formula (II)

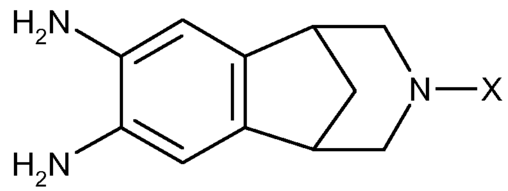

(II)

wherein X is an amino protecting group, and wherein the cyclization is carried out in the presence of a biphasic solvent system comprising an aqueous phase and an organic phase, so as to obtain a compound of formula (III)

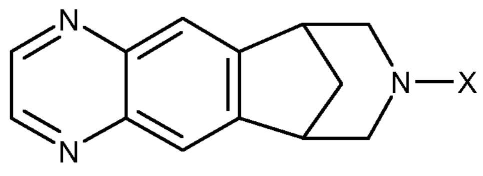

(III)

and treating the compound of formula (III) with an amino deprotecting agent suitable for removing the amino protecting group of the compound of formula (III), so as to yield varenicline, or a salt or solvate thereof, which deprotection is optionally carried out in the presence of a biphasic solvent system comprising an aqueous phase and an organic phase.

In a particularly preferred embodiment, the present invention provides a one-pot process of preparing varenicline, or a salt or solvate thereof, which process comprises cyclizing a compound of formula (II)

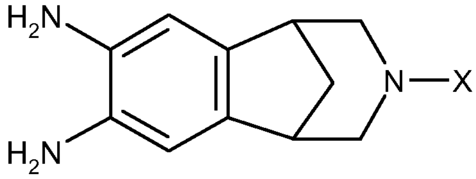

(II)

wherein X is an amino protecting group, so as to obtain a compound of formula (III)

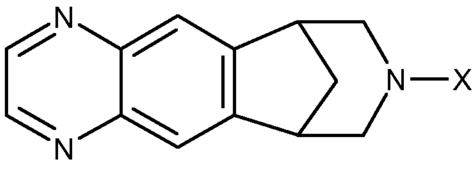

(III)

and treating the compound of formula (III) with an amino deprotecting agent suitable for removing the amino protecting group of the compound of formula (III), so as to yield varenicline, or a salt or solvate thereof,
characterized in that the cyclization and deprotection are carried out as a one-pot process in the presence of a common biphasic solvent system comprising an aqueous phase and an organic phase.

Suitably, the amino deprotecting agent employed so as to yield varenicline, or a salt or solvate thereof, comprises an organic or inorganic base and typically is selected from the group consisting of an alkali metal hydroxide, an alkali metal carbonate, an alkali metal hydrogencarbonate, an alkaline earth metal hydroxide, an alkaline earth metal carbonate, an alkaline earth metal hydrogencarbonate, an alkaline earth metal oxide, an ammonium hydroxide, an ammonium carbonate, an ammonium hydrogencarbonate, an amine and mixtures thereof. Preferably, the amino deprotecting agent employed is selected from the group consisting of sodium hydroxide, sodium hydrogencarbonate, sodium carbonate, potassium hydroxide, potassium hydrogencarbonate, potassium carbonate, calcium hydroxide, calcium carbonate, calcium oxide, ammonium hydroxide, ammonium carbonate, ammonium hydrogencarbonate, and mixtures thereof.

It is further preferred that the cyclizing agent to use in the process of the invention substantially as hereinbefore described is glyoxal or a glyoxal derivative, for example an aqueous glyoxal or a glyoxal derivative which is glyoxal sodium bisulfite addition compound hydrate. As referred to herein, aqueous glyoxal typically denotes a glyoxal / water ratio of about 95-5%, preferably a glyoxal / water ratio of about 75-15%, and more preferably a glyoxal / water ratio of about 40-20%. Additionally, it is preferred that the equivalent amount of the cyclizing agent to use in the process of the invention substantially as hereinbefore described is between 1.50-1.00, preferably between 1.20-1.00, and more preferably between 1.05 and 1.00, with respect to compound of formula (IIA).

The inventors of the present invention have thus unexpectedly found that cyclization of a compound of formula (II) can be carried out efficiently in the presence of an aqueous / organic biphasic system. This result was not predictable based on the prior art and the properties of the intermediate and reagent compounds employed. This is because a compound of formula (II) is a hydrophobic substance [for example, a compound of formula (IIa) shows an octanol / water partition coefficient (log P) of 1.33 (i.e. the concentration of a compound of formula (IIa) in octanol is more than 21 times higher than in water), as calculated using Advanced Chemistry Development (ACD/Labs) Software v8.14 for Solaris], whereas typically employed cyclization agents are hydrophilic substances [e.g. glyoxal shows an octanol / water logP value of - 0.85 (i.e., the concentration of glyoxal in water is more than 7 times higher than in octanol) See measured value on Concise International Chemical Assessment (CICAD) publication Document 57 (page 7, Table 1) from the International Programme on Chemical Safety (IPCS)]. On this basis, it could be expected that the cyclization in a biphasic solvent system would have much longer reaction time and much lower conversion, as compared with cyclization in the monophasic water / water miscible organic solvent media described in the processes of the prior art. Moreover, for water non-miscible organic solvents having a dielectric constant (ε) substantially lower than octanol (ε = 10.3) [e.g. toluene (ε = 2.4)], the differences in the solvent / water partition coefficient values between a compound of formula (II), and known cyclization reagents such as glyoxal, could be expected to increase with respect to those described for octanol, and thus the reaction could be expected to proceed at even slower rates and/or with lower conversions. Contrary to this

expectation, the inventors have found that cyclization of a compound of formula (II) in the presence of glyoxal sodium bisulfite addition compound hydrate or aqueous glyoxal and an aqueous / organic biphasic system, the reaction not only surprisingly proceeds efficiently and quantitatively (i.e. unreacted compound of formula (II) is not observed), but also can be carried out under mild temperature conditions that do not require heating or cooling steps (i.e. 20-25°C), and also can be carried out in shorter reaction times (i.e. 30 minutes), as compared with the water / water miscible organic solvent medium of the processes described in the prior art (i.e. 1 - 18 hours).

Furthermore, a process according to the present invention can provide still further advantages as compared with the processes described in the prior art.

For example, the inventors have surprisingly found that the by-product of formula (IVa) (also hereinafter more generally as a compound of formula (IV)) is formed in very low amounts and so a compound of formula (III) is obtained with a very high degree of chemical purity (i.e. about 99.6%, as measured by HPLC). Still further, a compound of formula (III) can be converted into varenicline directly, for example via a one-pot process, thus avoiding the prior art purification, isolation, and manipulation of a compound of formula (III) and also the prior art solvent replacement. Thus a process according to the present invention affords varenicline, or a salt or solvate thereof, in particular varenicline L-tartrate, with good yield as described herein. Additionally, the purification of a compound of formula (III), if any, can be carried out by means of simple liquid-liquid extraction procedures, without requiring the inefficient replacement of the water-miscible organic solvent and consequent addition of water-non miscible organic solvents to the reaction mixture which is described in the prior art.

In view of the above, it is further preferred that a process according to the present invention can additionally comprise carrying out at least one extraction purification step:

(a) for a compound of formula (III), or a salt or solvate thereof, prepared substantially as hereinbefore described; and / or

(b) for varenicline, or a salt or solvate thereof, prepared substantially as hereinbefore described.

Preferably, therefore, a process according to the present invention further comprises at least one extraction purification step comprising:

(a) directly isolating from a biphasic solvent system substantially as hereinbefore described either a compound of formula (III), or a salt or solvate thereof; or varenicline, or a salt or solvate thereof; and / or
(b) isolating either an aqueous or organic phase from a biphasic solvent system substantially as hereinbefore described, whereby the thus isolated aqueous or organic phase contains as appropriate the compound of formula (III), or a salt or solvate thereof; or as appropriate varenicline, or a salt or solvate thereof.

In certain embodiments, it is further preferred that step (b) above further comprises further isolating from an already isolated aqueous or organic phase, as appropriate a compound of formula (III), or a salt or solvate thereof, prepared substantially as hereinbefore described; or as appropriate varenicline, or a salt or solvate thereof, prepared substantially as hereinbefore described.

In certain embodiments, it is also further preferred that an at least one extraction purification step substantially as hereinbefore described further comprises carrying out at least one solvent washing step of an isolated aqueous or organic phase.

Isolation of either a compound of formula (III), or a salt or solvate thereof; or varenicline, or a salt or solvate thereof; as carried out in an at least one extraction purification step substantially as hereinbefore described, can typically comprise any suitable method known in the art, such as removing an organic solvent by distillation, or precipitating in an organic solvent by, for example, adding an anti-solvent.

As a way of example, the extraction purification step of compound of formula (III) or of varenicline can be carried out, for example, by partially distilling the solvent biphasic mixture to obtain a suspension comprising the compound, and filtering the compound, thereby directly isolating the compound from the biphasic solvent system. Alternatively, the distillation of the solvent mixture can be carried out until completion to obtain a residue comprising the compound, and treating the residue with a suitable solvent to obtain a suspension comprising the compound; filtering the compound; and optionally, preparing a new biphasic solvent system comprising the compound of formula (III) or a solvate thereof, or varenicline, or a solvate thereof, and where appropriate carrying out another extraction purification step or carrying out the next reaction step if the compound is compound of formula (III) or a solvate thereof. Also, the above extraction purification steps can further comprise a treatment of the biphasic solvent system, the suspension or the residue with a suitable inorganic or organic acid which may create acidic conditions suitable enough for obtaining and isolating compound of formula (III) or of varenicline in the form of the corresponding acid addition salt.

As an alternative example, the extraction purification step of compound of formula (III) or of varenicline can be

carried out, for example, by isolating from the biphasic solvent system the organic phase containing the compound of formula (III) or a solvate thereof, or varenicline, or a solvate thereof; optionally, carrying out one or more washings of the organic phase with an aqueous phase; optionally, isolating the compound from the organic phase by distilling or centrifuging the solvent, or by precipitating the product in the organic solvent by partially distilling off the solvent and/or cooling and/or adding an anti-solvent, and filtering the suspension; and optionally, preparing a new biphasic solvent system comprising the compound of formula (III) or a solvate thereof, or varenicline, or a solvate thereof, and where appropriate carrying out another extraction purification step or carrying out the next reaction step if the compound is compound of formula (III) or a solvate thereof.

Alternatively, the extraction purification step of compound of formula (III) or of varenicline can be carried out, for example, by treating the biphasic solvent system with a suitable inorganic or organic acid which may create acidic conditions suitable enough for converting compound of formula (III) or varenicline in the form of the corresponding acid addition salt; isolating from the aqueous solvent system the aqueous phase containing the acid addition salt of compound of formula (III) or of varenicline; optionally, carrying out one or more washings of the aqueous phase with an organic phase; optionally, isolating the compound from the aqueous phase by, for example, distilling or centrifuging the solvent, or by precipitating the product in the aqueous solvent by partially distilling off the solvent and/or cooling and/or adding an anti-solvent, and filtering the suspension; optionally converting the acid addition salt of compound (III) or of varenicline into compound of formula (III) or a solvate thereof, or varenicline, or a solvate thereof; and optionally, preparing a new biphasic solvent system comprising the compound of formula (III), or a salt or solvate thereof, or varenicline, or a salt or solvate thereof, and where appropriate carrying out another extraction purification step or carrying out the next reaction step if the compound is compound of formula (III) or a salt or solvate thereof.

The organic phase of a biphasic system as employed in a process according to the present invention substantially as hereinbefore described can comprise any organic solvent that is immiscible or at least not completely miscible with water. A water non-miscible organic solvent as referred to herein is understood to be an organic solvent that shows a reduced solubility in water and, therefore, separates as an upper or lower phase when the concentration of water is increased over its solubility limit. Preferred water non-miscible organic solvents are those having water solubility values (w/w) of less than 50%, more preferably less than 10%, even more preferably less than 1%, and even more preferably less than 0.1 %. Preferred suitable water non-miscible organic solvents to use in the process of the invention include $C_4$-$C_{10}$ alcohol solvent, $C_4$-$C_{10}$ ester solvent, $C_4$-$C_{10}$ ether solvent, $C_4$-$C_{10}$ ketone solvent, $C_1$-$C_{12}$ hydrocarbon solvent, $C_6$-$C_{12}$ aromatic solvent, and mixtures thereof. Non limiting examples of suitable water non-miscible organic solvents include pentyl acetate, tert-pentyl alcohol, anisole, benzene, benzyl alcohol, bromobenzene, 1-butanol, 2-butanol, butyl acetate, butyl ether, chlorobenzene, chloroform, cyclohexane, cyclohexanol, cyclohexanone, cyclopentane, cyclopentyl methyl ether, 1,2-dichlorobenzene, 1,2-dichloroethane, dichloromethane, diethoxymethane, 2-(2-hexylethoxy)ethanol, diisobutyl ketone, dimethoxymethane, ethyl acetate, ethylbenzene, 1,2-diethoxyethane, ethyl ether, n-heptane, n-hexane, 1-hexanol, isoamyl alcohol, isobutanol, isobutyl acetate, isopropyl acetate, isopropyl ether, methyl acetate, methyl tert-butyl ether, methylcyclohexane, methyl ethyl ketone, methyl formate, methyl isobutyl ketone, 2-methyltetrahydrofuran, nitrobenzene, 1-octanol, n-pentane, 1-pentanol, 3-pentanone, propyl acetate, propylene carbonate, 1-methoxy-2-propanol acetate, propylene oxide, tetrachloroethylene, toluene, 1,1,1-trichloroethane, trichloroethylene, xylene, and mixtures thereof. Particularly preferred organic solvents to form a biphasic system as employed in a process according to the present invention are selected from the group consisting of 1-butanol, 2-butanol, ethyl acetate, isopropyl acetate, methyl tert-butyl ether, methyl ethyl ketone, methyl isobutyl ketone, toluene, xylene, and mixtures thereof. A particularly preferred organic solvent for use in the present invention to form a biphasic system is toluene. In some embodiments, it may also be preferred that a biphasic system as employed in a process according to the present invention further comprises a water miscible organic solvent in an amount such that the aqueous / organic mixture is still a biphasic system.

The biphasic solvent system substantially as hereinbefore described preferably comprises an aqueous phase / organic phase substantially as hereinbefore described in a ratio of 0.1:99.9 (v/v) to 90.0:10.0 (v/v), preferably in a ratio of 1.0:99.0 (v/v) to 50.0:50.0 (v/v), and more preferably in a ratio 3.0:97.0 (v/v) to 10:90 (v/v).

A process according to the present invention substantially as hereinbefore described is preferably carried out under mild temperature conditions, preferably is carried out at a temperature between 0-50°C, more preferably is carried out at a temperature between 10-40°C, and more preferably is carried out at a temperature between 20-30°C.

A process according to the present invention substantially as hereinbefore described is preferably carried out in reduced reaction times, preferably is carried out in less than 6 hours, preferably is carried out in less than 1 hour, preferably is carried out in less than 45 minutes, and preferably is carried out in equal to or less than 30 minutes.

A process according to the present invention substantially as hereinbefore described can preferably further comprise decolorizing varenicline, or a salt or solvate thereof. Contrary to the teaching of the prior art wherein decolorizing of varenicline is always carried out in solvents with a high dielectric constant (i.e., $\varepsilon$ higher than 20), the inventors have surprisingly found that decolorizing of varenicline is particularly improved when carried out in organic solvent system with a low dielectric constant (i.e. $\varepsilon$ not higher than 20). More specifically, therefore, a process according to the present invention can further comprise the steps of:

(a) treating a solution of the hereinbefore prepared varenicline, or solvate thereof, in an organic solvent system having a dielectric constant not higher than 20, with a decolorizing agent;

(b) isolating varenicline, or solvate thereof, from the solution of step (a); and

(c) optionally and if appropriate, repeating steps (a) to (b) and / or converting varenicline obtained further to step (b) into a pharmaceutically acceptable salt or solvate.

The present invention also provides an independent process of decolorizing varenicline, or a salt or solvate thereof, which comprises the steps of:

(a) treating a solution of the varenicline, or solvate thereof, in an organic solvent system having a dielectric constant not higher than 20, with a decolorizing agent;

(b) isolating varenicline, or solvate thereof, from the solution of step (a); and

(c) optionally and if appropriate, repeating steps (a) to (b) and / or converting varenicline obtained further to step (b) into a pharmaceutically acceptable salt or solvate.

If appropriate, a decolorizing process according to the present invention can further comprise converting a varenicline salt into varenicline, or solvate thereof, prior to step (a).

An organic solvent as employed in step (a) of a decolorizing process according to the present invention preferably has a dielectric constant not higher than 10, and more preferably not higher than 7. Suitably, such an organic solvent as employed in step (a) of a decolorizing process according to the present invention comprises toluene, ethyl acetate, or mixtures thereof.

The decolorizing agent for use in a decolorizing process according to the present invention can be any suitable decolorizing agent known in the art. Preferably, however, the decolorizing agent is activated carbon.

A pharmaceutically acceptable salt of varenicline as prepared by any one process according to the present invention substantially as hereinbefore described can be any suitable salt of varenicline known in the art. Preferably, however, the pharmaceutically acceptable salt of varenicline is varenicline L-tartrate salt.

Substantially as hereinbefore described, a process according to the present invention affords varenicline, or a salt or solvate thereof, in particular varenicline L-tartrate, with good yield. According to the present invention, therefore, there is still further provided a process of preparing varenicline L-tartrate from a compound of formula (II)

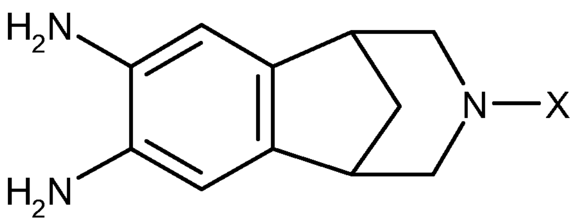

(II)

wherein X is an amino protecting group, wherein the varenicline L-tartrate is prepared in a percentage yield of at least 60%, and preferably is prepared in a percentage yield of at least 80%.

An amino protecting group as present in a compound of formula (II) or (III) substantially as hereinbefore described can be any known amino protecting group suitable for the protection of the amino group of compounds of formula (II) or (III), and is preferably selected from the group consisting of a trifluoroacetyl group, an acetyl group and a tert-butoxy carbonyl group. Most preferably, a compound of formula (II) substantially as hereinbefore described is a compound of formula (IIa), namely 10-(trifluoroacetyl)-10-azatricyclo[6.3.1.0$^{2,7}$]dodeca-2,4,6-triene-4,5-diamine

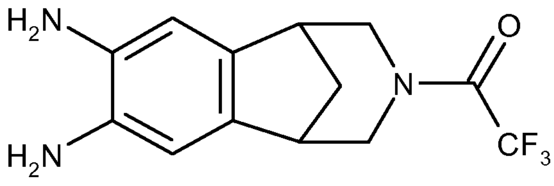

(IIa)

Most preferably, a compound of formula (III) substantially as hereinbefore described is a compound of formula (IIIa), namely 8-(trifluoroacetyl)-7,8,9,10-tetrahydro-6*H*-6,10-methanoazepino[4,5-g]quinoxaline

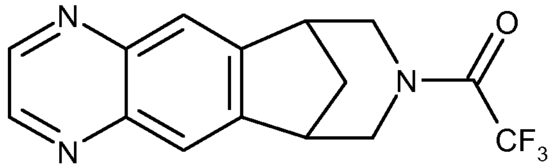

(IIIa)

The inventors have also found that a compound of formula (V), or a salt or solvate thereof

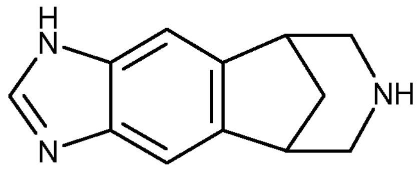

(V)

is formed as a low level impurity (V) in a process according to the present invention. A compound of formula (V) is known from U.S. Patent No. 6,410,550, but as an alternative active ingredient to varenicline, not as an impurity of varenicline. So, it is described that the compound of formula (V) has pharmacological activity. It is important in the pharmaceutical field that the API is administrated in a pure form and especially from those impurities, which, due to their pharmacological activity, may entail side effects. The identification of the impurity (V) is of a great significance, as it makes possible for the first time to provide a varenicline with a controlled level of such pharmacological active impurity.

Of particular importance in a process according to the present invention is the provision of an intermediate biphasic solvent system at least during cyclization of a compound of formula (II) so as to provide a compound of formula (III) substantially as hereinbefore described, and in a preferred one-pot process of preparing varenicline also the provision of an intermediate biphasic solvent system during deprotection so as to yield varenicline, or a salt or solvate thereof, again substantially as hereinbefore described. Accordingly, there is still further provided by the present invention an intermediate composition in the form of a biphasic solvent system, comprising an aqueous phase and an organic phase, which is formed further to cyclization of a compound of formula (II) substantially as hereinbefore described. Such an intermediate composition further comprises a compound of formula (III), or a salt or solvate thereof

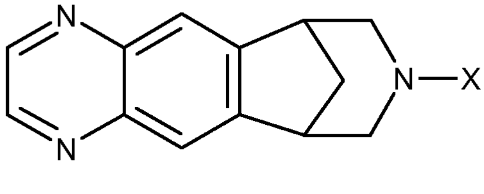

(III)

wherein X is an amino protecting group, and typically also comprises a compound of formula (IV), or a salt or solvate thereof

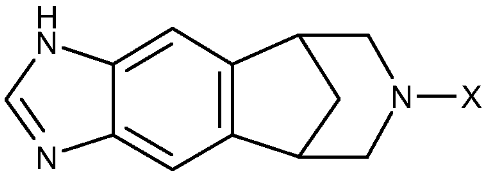

(IV)

wherein X is an amino protecting group. A compound of formula (IV) is a generic formula based on the known specific compound of formula (IVa) substantially as hereinbefore described with reference to the prior art. Typically, the above intermediate composition formed further to cyclization of a compound of formula (II) comprises a compound of formula (III) present in an amount of at least 99.50%, as measured by HPLC, and typically also a compound of formula (IV) present in an amount of less than 0.50%, as measured by HPLC.

Furthermore, with reference to a preferred one-pot process of preparing varenicline and a biphasic solvent system as employed in the deprotection of a compound of formula (III) substantially as hereinbefore described, there is additionally provided a further intermediate composition in the form of a biphasic solvent system comprising an aqueous phase and an organic phase, and which further comprises:

varenicline of formula (I), or a salt or solvate thereof

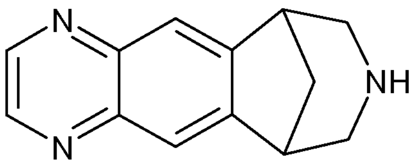

(I)

a compound of formula (V), or a salt or solvate thereof

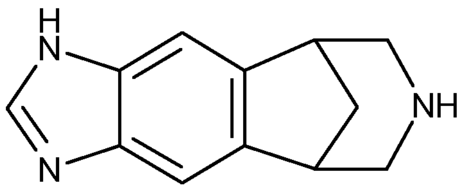

(V)

optionally, a compound of formula (III), or a salt or solvate thereof

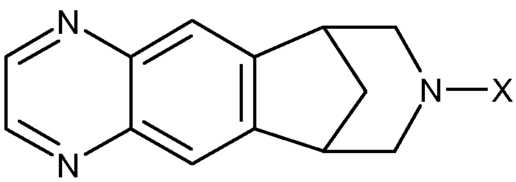

(III)

and, optionally, a compound of formula (IV), or a salt or solvate thereof

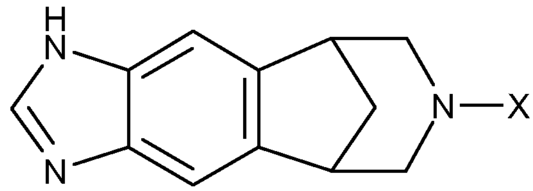

(IV)

wherein X is an amino protecting group. Typically, the above intermediate composition formed further to deprotection of a compound of formula (III) comprises varenicline present in an amount of at least 99.50%, as measured by HPLC, a compound of formula (V) present in an amount of less than 0.15%, as measured by HPLC, typically a compound of formula (III) present in an amount of less than 0.15%, as measured by HPLC and typically a compound of formula (IV) present in an amount of less than 0.15%, as measured by HPLC.

In the case where an intermediate composition according to the present invention includes a compound of formula (III), this is preferably a compound of formula (IIIa)

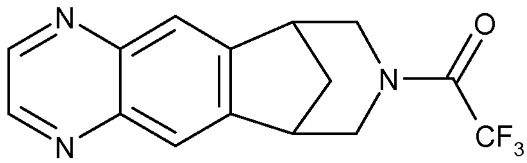

(IIIa)

and where an intermediate composition according to the present invention includes a compound of formula (IV), this is preferably a compound of formula (IVa)

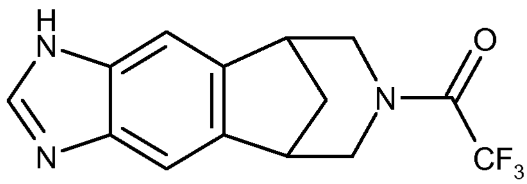

(IVa)

Typically, for an intermediate composition according to the present invention the organic phase comprises at least one solvent selected from the group consisting of 1-butanol, 2-butanol, ethyl acetate, isopropyl acetate, methyl tert-butyl ether, methyl ethyl ketone, methyl isobutyl ketone, toluene and xylene, preferably toluene.

Substantially as hereinbefore described, the inventors have found that a compound of formula (V), or a salt or solvate thereof, is formed as a low level impurity (V) in a process according to the present invention. There is further provided, therefore, use of a compound of formula (V), or a salt or solvate thereof

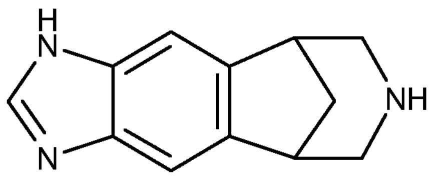

(V)

as a reference marker to analyze the purity of a sample comprising varenicline, or a salt or solvate thereof; and / or

as a reference standard to quantify the amount of a compound of formula (V) in a sample comprising varenicline, or a salt or solvate thereof; and / or
as a reference standard to ensure the purity of a sample comprising varenicline, or a salt or solvate thereof.

Furthermore, there is provided a method of using a compound of formula (V), or a salt or solvate thereof

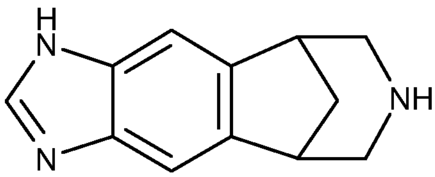

(V)

as a reference marker to analyze the purity of a test sample comprising varenicline, or a salt or solvate thereof. Typically, such a method comprises determining the presence of a compound of formula (V), or a salt or solvate thereof, in a test sample comprising varenicline, or a salt or solvate thereof, preferably by carrying out the steps of:

(a) providing a reference sample comprising (i) varenicline, or a salt or solvate thereof, and (ii) a reference marker which is a compound of formula (V), or a salt or solvate thereof;
(b) carrying out chromatographic separation on the reference sample to obtain a reference chromatographic result of the reference marker relative to varenicline, or a salt or solvate thereof;
(c) carrying out chromatographic separation on the test sample to obtain a test chromatographic result;
(d) comparing the chromatographic results obtained in steps (b) and (c); wherein if the test chromatographic result is substantially the same as the reference chromatographic result for the reference marker, then a compound of formula (V), or a salt or solvate thereof, is present in the test sample.

Preferably, the chromatographic separation comprises HPLC or GC and as such the above method comprises carrying out the steps of:

(a) providing a reference sample comprising (i) varenicline, or a salt or solvate thereof, and (ii) a reference marker which is a compound of formula (V), or a salt or solvate thereof;
(b) carrying out HPLC or GC on the reference sample to determine the relative retention time of the reference marker compared to varenicline, or a salt or solvate thereof;
(c) carrying out HPLC or GC on the test sample;
(d) comparing relative retention times determined in steps (b) and (c); wherein if there is observed a relative retention time in step (c) substantially the same as the relative retention time of the reference marker compared to varenicline, or a salt or solvate thereof, in step (b), then a compound of formula (V), or a salt or solvate thereof, is present in the test sample.

Alternatively, the chromatographic separation can comprise TLC and as such the above method comprises carrying out the steps of:

(a) providing a reference sample comprising (i) varenicline, or a salt or solvate thereof, and (ii) a reference marker which is a compound of formula (V), or a salt or solvate thereof;
(b) carrying out TLC on the reference sample to determine the relative component position on a chromatographic support of the reference marker compared to varenicline, or a salt or solvate thereof;
(c) carrying out TLC on the test sample;
(d) comparing the relative component positions determined in steps (b) and (c); wherein if there is observed on the chromatographic support a relative component position in step (c) substantially the same as the relative component position of the reference marker compared to varenicline, or a salt or solvate thereof, in step (b), then a compound of formula (V), or a salt or solvate thereof, is present in the test sample.

The present invention still further comprises a method of using a compound of formula (V), or a salt or solvate thereof

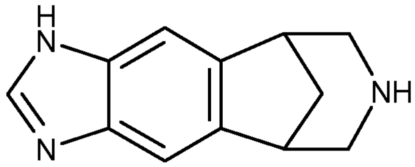

(V)

as a reference standard to quantify the amount of a compound of formula (V), or a salt or solvate thereof, in a test sample comprising varenicline, or a salt or solvate thereof. Typically, this quantification method comprises:

(a) providing a test sample of varenicline, or a salt or solvate thereof, containing an unknown concentration of a compound of formula (V), or a salt or solvate thereof;
(b) subjecting the test sample to chromatographic separation;
(c) obtaining a chromatographic quantitative measurement for a compound of formula (V), or a salt or solvate thereof, in the test sample; and
(d) calculating the amount of a compound of formula (V), or a salt or solvate thereof, in the test sample based on the measurement of step (c) and also chromatographic quantitative measurement for a compound of formula (V), or a salt or solvate thereof, obtained from at least one reference sample having a known concentration of a compound of formula (V), or a salt or solvate thereof.

In a first embodiment of the above quantification method, the following steps are carried out:

(a) providing a test sample of varenicline, or a salt or solvate thereof, containing an unknown concentration of a compound of formula (V), or a salt or solvate thereof;
(b) providing at least one reference sample having a known concentration of a compound of formula (V), or a salt or solvate thereof;
(c) subjecting the test sample and the reference sample to chromatographic separation;
(d) obtaining chromatographic quantitative measurements for a compound of formula (V), or a salt or solvate thereof, in the test sample and the reference sample; and
(e) calculating the amount of a compound of formula (V), or a salt or solvate thereof, in the test sample from the measurements of step (d).

More specifically, the following steps are typically carried out in the above first embodiment of the quantification method according to the present invention:

(a) providing a test sample of varenicline, or a salt or solvate thereof, containing an unknown concentration of a compound of formula (V), or a salt or solvate thereof;
(b) providing at least one reference sample having a known concentration of a compound of formula (V), or a salt or solvate thereof;
(c) subjecting the test sample and the reference sample to HPLC or GC;
(d) measuring the area or height of peaks obtained for a compound of formula (V), or a salt or solvate thereof, in the test sample and the reference sample; and
(e) calculating the concentration of a compound of formula (V), or a salt or solvate thereof, in the test sample from the measurements of step (d).

Alternatively, in a second embodiment of the above quantification method, the following steps are carried out:

(a) providing a test sample of varenicline, or a salt or solvate thereof, containing an unknown concentration of a compound of formula (V), or a salt or solvate thereof;
(b) subjecting the test sample to chromatographic separation;
(c) obtaining chromatographic quantitative measurement for a compound of formula (V), or a salt or solvate thereof, in the test sample; and
(d) calculating the concentration of a compound of formula (V), or a salt or solvate thereof, in the test sample based on the measurement of step (c) and also a calibration curve that is representative of chromatographic quantitative measurement for a compound of formula (V), or a salt or solvate thereof, obtained from more than one reference sample each having a respectively defined concentration of a compound of formula (V), or a salt or solvate thereof.

Typically, the calibration curve referred to above is obtained with at least two reference samples, preferably with at least five or six reference samples, and the concentration of a compound of formula (V), or a salt or solvate thereof, is then calculated by reference to the thus obtained calibration curve.

There is still further provided by the present invention a method of using a compound of formula (V), or a salt or solvate thereof

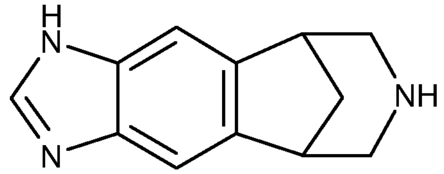

(V)

as a reference standard to ensure the purity of a test sample comprising varenicline, or a salt or solvate thereof. Typically, this purity method comprises carrying out a quantification method substantially as hereinbefore described so as to ensure that varenicline, or a salt or solvate thereof, as present in the test sample has less than 0.15% as measured by HPLC of a compound of formula (V), or a salt or solvate thereof.

There is further provided by the present invention a process for producing a validated batch of varenicline, or a pharmaceutical composition comprising thereof substantially as hereinbefore described, said process comprising:

a) producing a batch of varenicline, or a pharmaceutical composition comprising thereof;
b) determining the total amount of compound of formula (V), or a salt or solvate thereof,

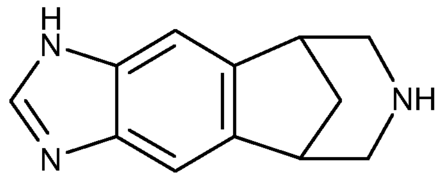

(V)

in a sample of the batch; and
c) validating the batch for distribution only if the sample of the batch contains a suitable concentration of a compound of formula (V), or a salt or solvate thereof, preferably a concentration of less than 0.15%, preferably less than 0.10%, and preferably less than 0.05%, as measured by HPLC, with respect to varenicline. Preferably, the determining the total amount of compound of formula (V), or a salt or solvate thereof, of step b) is carried out through the quantification method of the invention described above.

There is also provided by the present invention varenicline, or a salt or solvate thereof, having less than 0.15%, preferably less than 0.10%, more preferably less than 0.05%, as measured by HPLC of a compound of formula (V), or a salt or solvate thereof

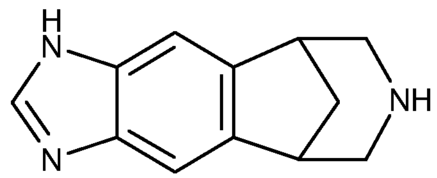

(V)

There is still further provided by the present invention varenicline, or a salt or solvate thereof, having between 0.10% and 0.15% as measured by HPLC of a compound of formula (V), or a salt or solvate thereof.

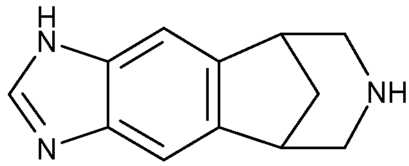

(V)

Preferably, the varenicline, or a salt or solvate thereof, according to the invention substantially as hereinbefore described shows enhanced color purity, meaning that it shows a white color substantially as described herein. Preferably, the varenicline, or a salt or solvate thereof, according to the invention shows the following measurements in the CIE (1976) L*, a*, b* Color Space (CIELAB) when using a colorimeter or spectrophotometer, illuminant D65 and a 2° angle of observation:

a) L* color coordinate value between 2 and 100
b) a* color coordinate value between -3.00 and +3.00; and
c) b* color coordinate value between -20.00 and +20.00.

Preferably, the varenicline, or a salt or solvate thereof, according to the invention substantially as hereinbefore described shows a Whiteness Index higher than 50.00, preferably higher than 75.00, preferably higher than 90.00, when using a colorimeter or spectrophotometer, illuminant D65 and a 2° angle of observation.

Accordingly, the present invention further provides a pharmaceutical composition comprising an effective amount of varenicline substantially as hereinbefore described, together with a pharmaceutically acceptable excipient. The term "effective amount" as used herein means an amount of varenicline which is capable of preventing, ameliorating or eliminating smoking addiction. By "pharmaceutically acceptable composition" is meant that the carrier, diluent or excipient must be compatible with varenicline and not be deleterious to a recipient thereof. Suitable pharmaceutically acceptable compositions according to the present invention are preferably for oral administration, and a particularly preferred oral dosage form comprises film coated tablets of varenicline.

Still further, the present invention provides varenicline substantially as hereinbefore described for use in treating smoking cessation.

Still further, there is provided a method of treating smoking cessation, which comprises administration of varenicline substantially as hereinbefore described to a subject.

All documents referred to herein, including patents, patent applications, and printed publications, are hereby incorporated by reference in their entirety in this disclosure.

The present invention will now be further illustrated by reference to the following Examples, which do not limit the scope of the invention in any way. Parts and percentages being by weight, unless otherwise indicated.

EXAMPLES

General Experimental Conditions

HPLC method:

The chromatographic separation was carried out in a Waters Atlantis dC18, 5μm, 4.6 x 150mm column. The mobile phase A was water (0.1% trifluoroacetic acid) and the mobile phase B was methanol. The chromatograph was equipped with a 230nm detector.

The chromatograph was programmed as follows: 0-17 minutes isocratic 78% mobile phase A and 22% mobile phase B; 17-37 minutes linear gradient to 60% mobile phase A; 37-65 minutes isocratic 60% mobile phase A; 65-70 minutes linear gradient to 78% mobile phase A; 70-80 minutes equilibration with 78% mobile phase A. The flow rate is 1 mL per minute at 30°C. Test samples (10μL) were prepared dissolving the appropriate amount of sample to obtain a concentration of 0.5mg of sample per mL of diluent [mixture of methanol and water (0.1 % trifluoroacetic acid) (25:75, v/v)].

Colorimetric measurement:

Colorimetric measurements of the solid samples were obtained using a Minolta Chroma meter CR-300, using illuminant D65 and a measurement geometry of 2°.

The color quality (i.e., "whiteness") of the varenicline L-tartrate samples was measured by depositing, leveling

and measuring the sample without any special compacting treatment. Measurement was repeated three times for each sample. For characterization, the values of L*, a*, b* and Whiteness Index (WI) were specifically listed, each one being the mean of the three values available for each characterization parameter.

Thus, the color of the solid sample is located in the CIE 1976 L*, a*, b* Color Space (CIELAB; CIE stands for Commission Internationale de l'Éclairage or International Commission on Illumination). The three parameters in the model represent the lightness of the color (i.e., L*, an L* = 0 indicates black and L* = 100 indicates white), its position between magenta and green (i.e., a*, negative values indicate green while positive values indicate magenta) and its position between yellow and blue (i.e., b*, negative values indicate blue and positive values indicate yellow). The color quality (i.e., "whiteness") of varenicline L-tartrate is corroborated by the values in the CIELAB color space. This can be further understood by reference to the US Pharmacopoeia 32nd ed., General Chapter 1061, p. 512.

The Whiteness Index (WI) of varenicline L-tartrate is calculated according to ASTM E313-05 "Standard Practice for Calculating Yellowness and Whiteness Indices from Instrumentally Measured Color Coordinates" using the following formula:

$$WI = Y + (WI_{,x})(x_n - x) + (WI_{,y})(y_n - y)$$

where: $x_n$ and $y_n$ are the chromaticity coordinates for the CIE Standard illuminant and source used, $WI_{,x}$ and $WI_{,y}$ are numerical coefficients, and Y, x, and y are the luminance factor and the chromaticity coordinates of the specimen (which can be derived from the L*, a*, and b* coordinates for a given illuminant and measurement geometry). Values for all these variables (except those measured for the specimen), for illuminant D65 (daylight) and a 2° angle of observation, are provided in Table 1.

Table 1

| | |
|---|---|
| $x_n$ | 0.3127 |
| $y_n$ | 0.3290 |
| $WI_{,x}$ | 800 |
| $WI_{,y}$ | 1700 |

A perfect white is a Perfect Reflecting Diffuser (PRD), which has a Whiteness Index value of 100. Pressed magnesium oxide (MgO) and pressed barium sulfate ($BaSO_4$) are high-reflectance materials that closely approximate a PRD.

Example 1: Preparation of 7,8,9,10-tetrahydro-6,10-methano-6*H*-pyrazino[2,3-h][3]benzazepine L-tartrate (i.e. varenicline L-tartrate)

This example illustrates a one-pot preparation of Varenicline L-tartrate from a compound of formula of formula (IIa) in an aqueous / organic biphasic system according to a process of the invention. Also, this example illustrates the decolorization of varenicline L-tartrate according to a process of the invention.

Step A: Preparation of 8-(trifluoroacetyl)-7,8,9,10-tetrahydro-6*H*-6,10-methanoazepino[4,5-*g*]quinoxaline (a compound of formula IIIa) from 10-(trifluoroacetyl)-10-azatricyclo[6.3.1.0$^{2,7}$]dodeca-2,4,6-triene-4,5-diamine (a compound of formula IIa)

A 5L reactor equipped with thermometer, condenser and mechanical stirring was charged with 10-(trifluoroacetyl)-10-azatricyclo[6.3.1.0$^{2,7}$]dodeca-2,4,6-triene-4,5-diamine (200g, 701 mmol) [i.e. a compound of formula (IIa)] and toluene (2.00L). To this suspension was charged an aqueous saturated solution of sodium bicarbonate (34.9mL). To the aqueous / organic biphasic mixture was slowly added an aqueous solution of glyoxal 40% (84.43mL, 736mmol). The resulting aqueous / organic biphasic mixture was stirred at 20-25°C for 30 minutes [a sample was taken and analyzed by HPLC: a compound of formula (IIIa) (99.629%), a compound of formula (IVa) (0.326%)].

Step B: Preparation of varenicline L-tartrate from 8-(trifluoroacetyl)-7,8,9,10-tetrahydro-6H-6,10-methanoazepino[4,5-*g*]quinoxaline (a compound of formula IIIa)

To the aqueous / organic biphasic mixture of step A was charged a 2N aqueous solution of sodium hydroxide

(1.23L, 2.45mol), which was heated up to 40-45°C. The biphasic system was stirred at this temperature for 2 hours [a sample was taken and analyzed by HPLC: a compound of formula (I) (98.149%), a compound of formula (IIIa) (0.535%), a compound of formula (IVa) (0.237%), a compound of formula (V) (0.098%)]. The stirring was stopped and the layers were let to decant at 40-45°C. The layers were separated and the aqueous layer was extracted with toluene (2 x 1.00L). The combined organic layers were evaporated to dryness at 40°C under vacuum. The residue was dissolved in methanol (740mL) and a solution of L-tartaric acid (105g, 701 mmol) in methanol (740mL) was slowly added. The suspension was stirred at 20-25°C for 1 hour, filtered, washed with methanol (2 x 200mL) and dried at 40°C under vacuum, to obtain 224g (88% yield) of Varenicline L-tartrate as an orange solid [purity 99.813% by HPLC, a compound of formula (V) (0.039%)].

Step C: Decolorization of varenicline L-tartrate

A 5L reactor equipped with thermometer, condenser and mechanical stirring was charged with Varenicline L-tartrate (previous solid, 223g, 618mmol), toluene (2.23L) and water (1.12L). To the mixture was added 50% aqueous solution of sodium hydroxide (218mL, 4.13mol) and it was heated up to 40-45°C. The stirring was stopped and the layers were let to decant at 40-45°C. The layers were separated and the aqueous layer was extracted with toluene (1.12L). The combined organic layers were evaporated to dryness at 40°C under vacuum. The residue was dissolved in methanol (750mL) and activated charcoal (11.0g) was charged to the solution. The suspension was stirred at 20-25°C for 1 hour, filtered through a pad of Celite™ and washed with methanol (3 x 130mL). The solution was slowly added to a solution of L-tartaric acid (92.8g, 618mmol) in methanol (655mL). The suspension was stirred at 20-25°C for 1.5 hour, filtered, washed with methanol (2 x 100mL) and dried at 40°C under vacuum, to obtain 215g (96% yield) of a slightly orange solid [L* = 82.53, a* = 3.91, b* = 31.13; Whiteness Index -94.64] [purity 99.927% by HPLC, compound of formula (V) (0.014%)].

Example 2: Preparation of 7,8,9,10-tetrahydro-6,10-methano-6*H*-pyrazino[2,3-*h*][3]benzazepine L-tartrate (i.e. varenicline L-tartrate)

This example illustrates a one-pot preparation of Varenicline L-tartrate from a compound of formula (IIa) in an aqueous / organic biphasic system according to a process of the invention. Also, this example illustrates the decolorization of varenicline L-tartrate according to a process of the invention.

Step A: Preparation of 8-(trifluoroacetyl)-7,8,9,10-tetrahydro-6*H*-6,10-methanoazepino[4,5-*g*]quinoxaline (a compound of formula IIIa) from 10-(trifluoroacetyl)-10-azatricyclo[6.3.1.$0^{2,7}$]dodeca-2,4,6-triene-4,5-diamine (a compound of formula IIa)

A 25L reactor equipped with thermometer, condenser and mechanical stirring was charged with 10-(trifluoroacetyl)-10-azatricyclo[6.3.1.$0^{2,7}$]dodeca-2,4,6-triene-4,5-diamine (1.28Kg, 4.49mol) [i.e. a compound of formula (IIa)] and toluene (13.2L). To this suspension was charged a solution of sodium bicarbonate (20.0g) in water (184mL). To the aqueous / organic biphasic mixture was slowly added an aqueous solution of glyoxal 40% (0.54L, 4.67mol). The resulting aqueous / organic biphasic mixture was stirred at 20-25°C for 30 minutes.

Step B: Preparation of varenicline L-tartrate from 8-(trifluoroacetyl)-7,8,9,10-tetrahydro-6H-6,10-methanoazepino[4,5-*g*] quinoxaline (a compound of formula IIIa)

To the aqueous / organic biphasic mixture of step A was charged a solution of 50% aqueous solution of sodium hydroxide (1.27Kg, 15.8mol) in water (7.02L) which was heated up to 40-45°C. The biphasic system was stirred at this temperature for 2 hours. The stirring was stopped and the layers were let to decant at 40-45°C. The layers were separated and the aqueous layer was extracted with toluene (2 x 6.40L). The combined organic layers were evaporated to dryness at 40°C under vacuum. The residue was dissolved in methanol (5.36L) and a solution of L-tartaric acid (669g, 4.46mol) in methanol (4.72L) was slowly added. The suspension was stirred at 20-25°C for 1 hour, filtered and washed with methanol (2 x 1.26L) to obtain 2.33Kg (l.o.d.: 37.88%, 89% yield) of Varenicline L-tartrate as an orange solid [purity 99.251% by HPLC, a compound of formula (V) (0.025%)].

Step C: Decolorization of varenicline L-tartrate

A 25L reactor equipped with thermometer, condenser and mechanical stirring was charged with Varenicline L-tartrate (previous wet solid), toluene (13.5L) and water (6.51 L). To the mixture was added 50% aqueous solution of sodium hydroxide (1.31 L, 24.9mol) and it was heated up to 40-45°C. The stirring was stopped and the layers were let

to decant at 40-45°C. The layers were separated and the aqueous layer was extracted with toluene (6.51 L). To the combined organic layers activated charcoal (63g) was added. The suspension was stirred at 20-25°C for 1 hour, filtered through a pad of Celite™ and washed with toluene (2 x 0.70L). The filtrate was evaporated to dryness at 40°C under vacuum. The residue was dissolved in methanol (4.84L) and the solution was slowly added to a solution of L-tartaric acid (535g, 3.56mol) in methanol (3.20L). The suspension was stirred at 20-25°C for 1 hour, filtered, washed with methanol (2 x 0.32L) to obtain 2.51 Kg (l.o.d.: 46.98%, 82% yield) of Varenicline L-tartrate as a slightly yellow solid.

A 25L reactor equipped with thermometer, condenser and mechanical stirring was charged with Varenicline L-tartrate (previous wet solid), toluene (14.1L) and water (7.07L). To the mixture was added 50% aqueous solution of sodium hydroxide (0.72L, 13.6mol) and it was heated up to 40-45°C. The stirring was stopped and the layers were let to decant at 40-45°C. The layers were separated and the aqueous layer was extracted with toluene (7.24L). To the combined organic layers activated charcoal (71g) was added. The suspension was stirred at 20-25°C for 1 hour, filtered through a pad of Celite™ and washed with toluene (2 x 0.50L). The filtrate was evaporated to dryness at 40°C under vacuum. The residue was dissolved in methanol (4.73L) and the solution was slowly added to a solution of L-tartaric acid (594g, 3.96mol) in methanol (4.10L). The suspension was stirred at 20-25°C for 1 hour, filtered, washed with methanol (2 x 0.35L) and dried at 40°C under vacuum, to obtain 1.12Kg (84% yield) of Varenicline L-tartrate as an off-white solid [L* = 95.95, a* = 0.04, b* = 6.85; Whiteness Index: 59.04] [purity 99.656% by HPLC, a compound of formula (V) (0.007%)].

Examples 3 to 5: Decolorization of 7,8,9,10-tetrahydro-6,10-methano-6*H*-pyrazino[2,3-*h*][3]benzazepine L-tartrate (i.e. varenicline L-tartrate)

Step A: Decolorization of varenicline

General procedure for Examples 3 and 4: Varenicline (1.1g, 5.21mmol) was charged into a 50mL reactor and was dissolved in 25mL of solvent (see Table 2 for examples 3 and 4). Active charcoal (0.1 g) was charged to the solution and it was stirred at 20-25°C for 1 hour. The suspension was filtered through a pad of Celite™ and washed with 2 x 5mL of solvent (see Table 2). The mother liquors were evaporated to dryness under vacuum.

Step B: Preparation of varenicline L-tartrate

General procedure for Examples 3 to 5: The decolorized residue (from examples 3 and 4) or untreated Varenicline (1.1g, 5.21mmol, Example 5) was dissolved in methanol (9mL). The solution was slowly added to a solution of L-tartaric acid (0.86g, 5.73mmol) in methanol (9mL). The resulting suspension was stirred at 20-25°C for 1 hour, filtered, washed with methanol (2 x 1 mL) and dried at 40°C under vacuum to obtain Varenicline L-tartrate. The colorimetric results are shown in following Table 2. For Example 3, Varenicline L-tartrate was obtained in a percentage yield of 66.1 %.

Table 2

| Example | Solvent for the decolorization step A | ε | L* | a* | b* | Whiteness Index |
|---|---|---|---|---|---|---|
| 3 | Ethyl acetate | 6.0 | 93.85 | 0.81 | 13.21 | 24.27 |
| 4 | Methanol | 32.7 | 86.33 | 1.44 | 15.50 | -7.54 |
| 5 | - | - | 84.66 | 5.74 | 22.64 | -46.09 |

## Claims

**1.** A process of preparing a compound of formula (IIIA)

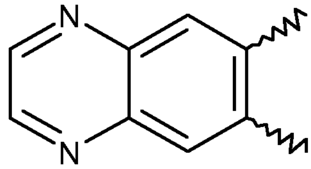

(IIIA)

said process comprising cyclizing a compound of formula (IIA)

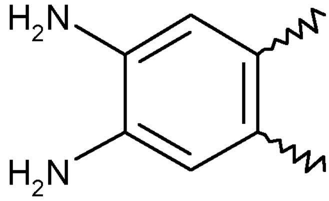

(IIA)

with a cyclizing agent, so as to obtain said compound of formula (IIIA),
**characterized in that** said cyclization is carried out in the presence of a biphasic solvent system comprising an aqueous phase and an organic phase.

2. A process according to claim 1, wherein said compound of formula (IIIA) is a compound of formula (III)

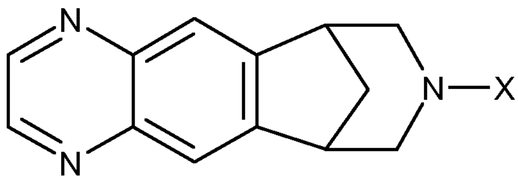

(III)

and said compound of formula (IIA) is a compound of formula (II)

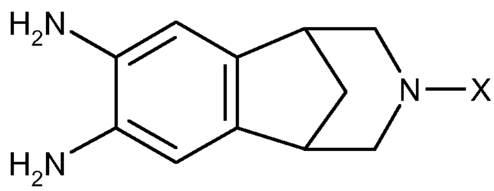

(II)

wherein X is an amino protecting group.

3. A process according to claim 2, which further comprises treating said compound of formula (III) with an amino deprotecting agent suitable for removing the amino protecting group of said compound of formula (III), so as to yield varenicline, or a salt or solvate thereof, which deprotection is optionally carried out in the presence of a biphasic solvent system comprising an aqueous phase and an organic phase.

4. A process according to claim 3, whereby varenicline, or a salt or solvate thereof, is prepared in a one-pot process from said compound of formula (II) via said compound of formula (III).

5. A one-pot process of preparing varenicline, or a salt or solvate thereof, which process comprises cyclizing a compound of formula (II)

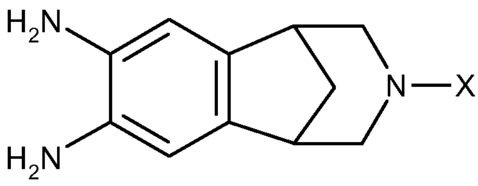

(II)

wherein X is an amino protecting group, so as to obtain a compound of formula (III)

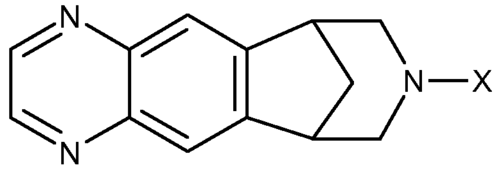

(III)

and treating said compound of formula (III) with an amino deprotecting agent suitable for removing the amino protecting group of said compound of formula (III), so as to yield varenicline, or a salt or solvate thereof, **characterized in that** said cyclization and deprotection are carried out as a one-pot process in the presence of a common biphasic solvent system comprising an aqueous phase and an organic phase.

**6.** A process according to any of claims 3 to 5, wherein the amino deprotecting agent is an organic or inorganic base.

**7.** A process according to claim 6, wherein the amino deprotecting agent is selected from the group consisting of an alkali metal hydroxide, an alkali metal carbonate, an alkali metal hydrogen carbonate, an alkaline earth metal hydroxide, an alkaline earth metal carbonate, an alkaline earth metal oxide, an ammonium hydroxide, an ammonium carbonate, an ammonium hydrogencarbonate, an amine and mixtures thereof.

**8.** A process according to any of claims 1 to 7, wherein said cyclizing agent is glyoxal or a glyoxal derivative.

**9.** A process according to claim 8, wherein said cyclization is carried out in the presence of aqueous glyoxal.

**10.** A process according to claim 8, wherein said cyclization is carried out in the presence of a glyoxal derivative which is glyoxal sodium bisulfite addition compound hydrate.

**11.** A process according to any of claims 2 to 10, which further comprises carrying out at least one extraction purification step:

(a) for a compound of formula (III), or a salt or solvate thereof, as obtained by a process according to any of claims 2 to 10; and / or
(b) for varenicline, or a salt or solvate thereof, as obtained by a process according to any of claims 3 to 10.

**12.** A process according to claim 11, wherein said at least one extraction purification step comprises:

(a) directly isolating from a biphasic solvent system as defined in any of claims 2 to 10 either a compound of formula (III), or a salt or solvate thereof, as obtained by a process according to any of claims 2 to 10; or varenicline, or a salt or solvate thereof, as obtained by a process according to any of claims 3 to 10; and / or
(b) isolating either an aqueous or organic phase from a biphasic solvent system as defined in any of claims 2 to 10, whereby said thus isolated aqueous or organic phase contains as appropriate said compound of formula (III), or a salt or solvate thereof, as obtained by a process according to any of claims 2 to 10; or as appropriate varenicline, or a salt or solvate thereof, as obtained by a process according to any of claims 3 to 10.

**13.** A process according to claim 12, wherein step (b) further comprises further isolating from said already isolated

aqueous or organic phase, as appropriate said compound of formula (III), or a salt or solvate thereof, as obtained by a process according to any of claims 2 to 10; or as appropriate varenicline, or a salt or solvate thereof, as obtained by a process according to any of claims 3 to 10.

**14.** A process according to claim 12, which further comprises carrying out at least one solvent washing step of said isolated aqueous or organic phase.

**15.** A process according to any of claims 1 to 14, wherein the organic phase comprises at least one solvent selected from the group consisting of $C_4$-$C_{10}$ alcohol solvent, $C_4$-$C_{10}$ ester solvent, $C_4$-$C_{10}$ ether solvent, $C_4$-$C_{10}$ ketone solvent, $C_1$-$C_{10}$ hydrocarbon solvent, $C_6$-$C_{12}$ aromatic solvent, and mixtures thereof, and preferably comprises at least one solvent selected from the group consisting of 1-butanol, 2-butanol, ethyl acetate, isopropyl acetate, methyl tert-butyl ether, methyl ethyl ketone, methyl isobutyl ketone, toluene, xylene, and mixtures thereof.

**16.** A process according to any of claims 3 to 15, which further comprises decolorizing varenicline, or a salt or solvate thereof.

**17.** A process according to claim 16, which comprises:

(a) treating a solution of the varenicline, or solvate thereof, in an organic solvent system having a dielectric constant not higher than 20, with a decolorizing agent;
(b) isolating varenicline, or solvate thereof, from the solution of step (a); and
(c) optionally and if appropriate, repeating steps (a) to (b) and / or converting varenicline obtained further to step (b) into a pharmaceutically acceptable salt or solvate.

**18.** A process of decolorizing varenicline, or a salt or solvate thereof, which comprises:

(a) treating a solution of the varenicline, or solvate thereof, in an organic solvent system having a dielectric constant not higher than 20, with a decolorizing agent;
(b) isolating varenicline, or solvate thereof, from the solution of step (a); and
(c) optionally and if appropriate, repeating steps (a) to (b) and / or converting varenicline obtained further to step (b) into a pharmaceutically acceptable salt or solvate.

**19.** A process according to claim 17 or 18, which further comprises converting a varenicline salt into varenicline, or solvate thereof, prior to step (a).

**20.** A process according to any of claims 17 to 19, wherein said organic solvent system has a dielectric constant not higher than 10.

**21.** A process according to claim 20, wherein said organic solvent system has a dielectric constant not higher than 7.

**22.** A process according to any of claims 17 to 21, wherein said organic solvent system comprises toluene, ethyl acetate, or mixtures thereof.

**23.** A process according to any of claims 17 to 22, wherein said decolorizing agent is activated carbon.

**24.** A process according to any of claims 3 to 23, wherein varenicline is prepared as varenicline L-tartrate.

**25.** A process of preparing varenicline L-tartrate from a compound of formula (II)

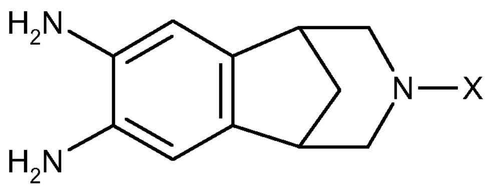

(II)

wherein X is an amino protecting group, wherein said varenicline L-tartrate is prepared in a percentage yield of at least 60%, and preferably is prepared in a percentage yield of at least 80%.

**26.** A process according to any of claims 2 to 17 or 25, wherein the amino protecting group is selected from the group consisting of a trifluoroacetyl group, an acetyl group and a tert-butoxy carbonyl group.

**27.** A process according to claim 26, wherein the amino protecting group is trifluoroacetyl, thereby a compound of formula (II) is a compound of formula (IIa), namely 10-(trifluoroacetyl)-10-azatricyclo[6.3.1.$0^{2,7}$]dodeca-2,4,6-triene-4,5-diamine

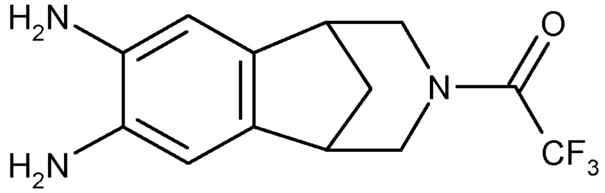

(IIa)

**28.** A process according to claim 27, as dependent on any of claims 2 to 17, wherein a compound of formula (III) is a compound of formula (IIIa), namely 8-(trifluoroacetyl)-7,8,9,10-tetrahydro-6*H*-6,10-methanoazepino[4,5-*g*]quinoxaline

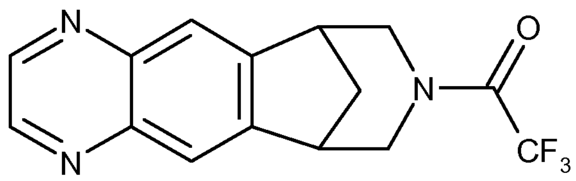

(IIIa)

**29.** An intermediate composition in the form of a biphasic solvent system comprising an aqueous phase and an organic phase, and which further comprises a compound of formula (III), or a salt or solvate thereof

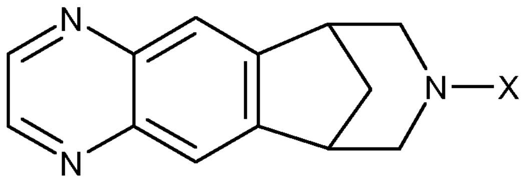

(III)

wherein X is an amino protecting group.

**30.** An intermediate composition according to claim 29, wherein said composition further comprises a compound of formula (IV), or a salt or solvate thereof

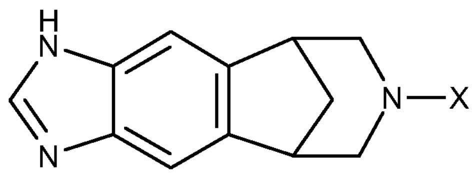

(IV)

wherein X is an amino protecting group.

**31.** An intermediate composition in the form of a biphasic solvent system comprising an aqueous phase and an organic phase, and which further comprises:

varenicline of formula (I), or a salt or solvate thereof

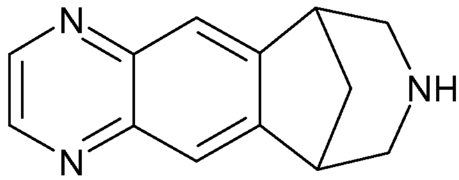

(I)

a compound of formula (V), or a salt or solvate thereof

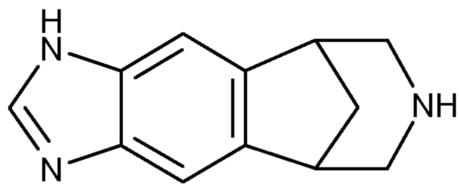

(V)

optionally, a compound of formula (III), or a salt or solvate thereof

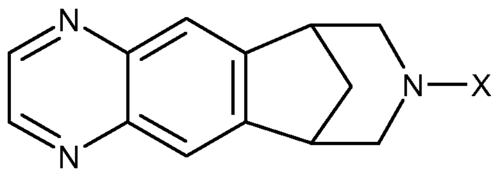

(III)

and, optionally, a compound of formula (IV), or a salt or solvate thereof

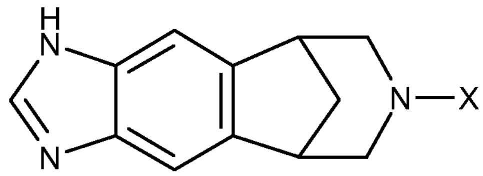

(IV)

wherein X is an amino protecting group.

**32.** An intermediate composition according to claim 29, wherein a compound of formula (III) is present in an amount of at least 99.50%, as measured by HPLC.

**33.** An intermediate composition according to claim 30, wherein a compound of formula (IV) is present in an amount of less than 0.50%, as measured by HPLC.

**34.** An intermediate composition according to claim 31, wherein varenicline is present in an amount of at least 99.50%, as measured by HPLC.

**35.** An intermediate composition according to claim 31, wherein a compound of formula (V) is present in an amount of less than 0.15%, as measured by HPLC.

**36.** An intermediate composition according to claim 31, wherein a compound of formula (III) is present in an amount of less than 0.15%, as measured by HPLC.

**37.** An intermediate composition according to claim 31, wherein a compound of formula (IV) is present in an amount of less than 0.15%, as measured by HPLC.

**38.** An intermediate composition according to claim 29 or 31, wherein a compound of formula (III) is a compound of formula (IIIa)

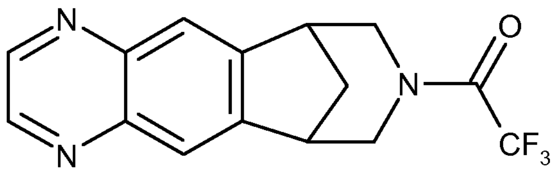

(IIIa)

**39.** An intermediate composition according to claim 30 or 31, wherein a compound of formula (IV) is a compound of formula (IVa)

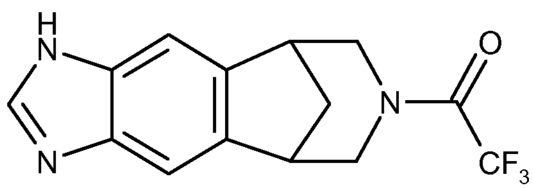

(IVa)

**40.** An intermediate composition according to any of claims 29 to 39, wherein the organic phase comprises at least one solvent selected from the group consisting of 1-butanol, 2-butanol, ethyl acetate, isopropyl acetate, methyl tert-butyl

ether, methyl ethyl ketone, methyl isobutyl ketone, toluene and xylene.

**41.** Use of a compound of formula (V), or a salt or solvate thereof

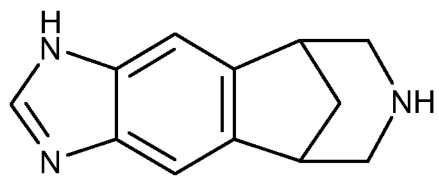

(V)

as a reference marker to analyze the purity of a sample comprising varenicline, or a salt or solvate thereof.

**42.** Use of a compound of formula (V), or a salt or solvate thereof

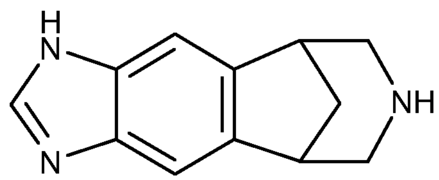

(V)

as a reference standard to quantify the amount of a compound of formula (V) in a sample comprising varenicline, or a salt or solvate thereof.

**43.** Use of a compound of formula (V), or a salt or solvate thereof

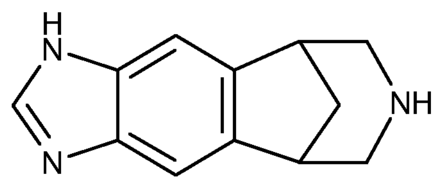

(V)

as a reference standard to ensure the purity of a sample comprising varenicline, or a salt or solvate thereof.

**44.** A method of using a compound of formula (V), or a salt or solvate thereof

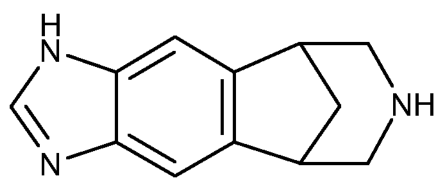

(V)

as a reference marker to analyze the purity of a test sample comprising varenicline, or a salt or solvate thereof.

**45.** Method for determining the presence of a compound of formula (V), or a salt or solvate thereof in a sample comprising varenicline, or a salt or solvate thereof, which comprises determining the presence of a compound of formula (V), or a salt or solvate thereof, in said sample comprising varenicline, or a salt or solvate thereof, using the marker described in claim 44.

**46.** A method according to claim 45, which comprises:

(a) providing a reference sample comprising (i) varenicline, or a salt or solvate thereof, and (ii) a reference marker which is a compound of formula (V), or a salt or solvate thereof;
(b) carrying out chromatographic separation on said reference sample to obtain a reference chromatographic result of said reference marker relative to said varenicline, or a salt or solvate thereof;
(c) carrying out chromatographic separation on said test sample to obtain a test chromatographic result;
(d) comparing the chromatographic results obtained in steps (b) and (c); wherein if the test chromatographic result is substantially the same as the reference chromatographic result for said reference marker, then a compound of formula (V), or a salt or solvate thereof, is present in said test sample.

**47.** A method according to claim 46, which comprises:

(a) providing a reference sample comprising (i) varenicline, or a salt or solvate thereof, and (ii) a reference marker which is a compound of formula (V), or a salt or solvate thereof;
(b) carrying out HPLC or GC on said reference sample to determine the relative retention time of said reference marker compared to said varenicline, or a salt or solvate thereof;
(c) carrying out HPLC or GC on said test sample;
(d) comparing relative retention times determined in steps (b) and (c); wherein if there is observed a relative retention time in step (c) substantially the same as the relative retention time of said reference marker compared to said varenicline, or a salt or solvate thereof, in step (b), then a compound of formula (V), or a salt or solvate thereof, is present in said test sample.

**48.** A method according to claim 46, which comprises:

(a) providing a reference sample comprising (i) varenicline, or a salt or solvate thereof, and (ii) a reference marker which is a compound of formula (V), or a salt or solvate thereof;
(b) carrying out TLC on said reference sample to determine the relative component position on a chromatographic support of said reference marker compared to said varenicline, or a salt or solvate thereof;
(c) carrying out TLC on said test sample;
(d) comparing the relative component positions determined in steps (b) and (c); wherein if there is observed on said chromatographic support a relative component position in step (c) substantially the same as the relative component position of said reference marker compared to said varenicline, or a salt or solvate thereof, in step (b), then a compound of formula (V), or a salt or solvate thereof, is present in said test sample.

**49.** A method of using a compound of formula (V), or a salt or solvate thereof

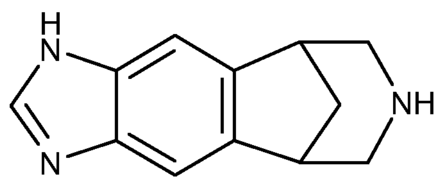

(V)

as a reference standard to quantify the amount of a compound of formula (V), or a salt or solvate thereof, in a test sample comprising varenicline, or a salt or solvate thereof.

**50.** A method according to claim 49, which comprises:

(a) providing a test sample of varenicline, or a salt or solvate thereof, containing an unknown concentration of

a compound of formula (V), or a salt or solvate thereof;
(b) subjecting said test sample to chromatographic separation;
(c) obtaining a chromatographic quantitative measurement for a compound of formula (V), or a salt or solvate thereof, in said test sample; and
(d) calculating the amount of a compound of formula (V), or a salt or solvate thereof, in said test sample based on the measurement of step (c) and also chromatographic quantitative measurement for a compound of formula (V), or a salt or solvate thereof, obtained from at least one reference sample having a known concentration of a compound of formula (V), or a salt or solvate thereof.

**51.** A method according to claim 50, which comprises:

(a) providing a test sample of varenicline, or a salt or solvate thereof, containing an unknown concentration of a compound of formula (V), or a salt or solvate thereof;
(b) providing at least one reference sample having a known concentration of a compound of formula (V), or a salt or solvate thereof;
(c) subjecting said test sample and said reference sample to chromatographic separation;
(d) obtaining chromatographic quantitative measurements for a compound of formula (V), or a salt or solvate thereof, in said test sample and said reference sample; and
(e) calculating the amount of a compound of formula (V), or a salt or solvate thereof, in said test sample from the measurements of step (d).

**52.** A method according to claim 51, which comprises:

(a) providing a test sample of varenicline, or a salt or solvate thereof, containing an unknown concentration of a compound of formula (V), or a salt or solvate thereof;
(b) providing at least one reference sample having a known concentration of a compound of formula (V), or a salt or solvate thereof;
(c) subjecting said test sample and said reference sample to HPLC or GC;
(d) measuring the area or height of peaks obtained for a compound of formula (V), or a salt or solvate thereof, in said test sample and said reference sample; and
(e) calculating the concentration of a compound of formula (V), or a salt or solvate thereof, in said test sample from the measurements of step (d).

**53.** A method according to claim 50, which comprises:

(a) providing a test sample of varenicline, or a salt or solvate thereof, containing an unknown concentration of a compound of formula (V), or a salt or solvate thereof;
(b) subjecting said test sample to chromatographic separation;
(c) obtaining chromatographic quantitative measurement for a compound of formula (V), or a salt or solvate thereof, in said test sample; and
(d) calculating the concentration of a compound of formula (V), or a salt or solvate thereof, in said test sample based on the measurement of step (c) and also a calibration curve that is representative of chromatographic quantitative measurement for a compound of formula (V), or a salt or solvate thereof, obtained from more than one reference sample each having a respectively defined concentration of a compound of formula (V), or a salt or solvate thereof.

**54.** A method of using a compound of formula (V), or a salt or solvate thereof

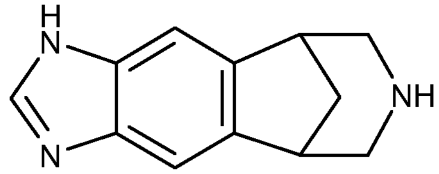

(V)

as a reference standard to ensure the purity of a test sample comprising varenicline, or a salt or solvate thereof.

**55.** A method for ensuring the purity of varenicline, or a salt or solvate thereof, in a sample comprising a compound of formula (V), or a salt or solvate thereof, which comprises carrying out a method according to any of claims 50 to 53, so as to ensure that varenicline, or a salt or solvate thereof, if it is present in said sample, has less than 0,15% as measured by HPLC of a compound of formula (V), or a salt or solvate thereof.

**56.** Varenicline, or a salt or solvate thereof, having less than 0.15% as measured by HPLC of a compound of formula (V), or a salt or solvate thereof

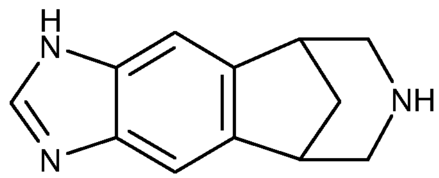

(V)

**57.** Varenicline, or a salt or solvate thereof, having less than 0.10% as measured by HPLC of a compound of formula (V), or a salt or solvate thereof

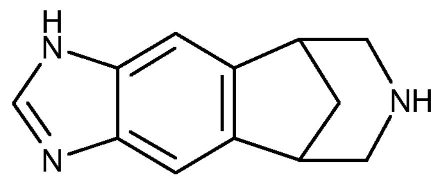

(V)

**58.** Varenicline, or a salt or solvate thereof, having less than 0.05% as measured by HPLC of a compound of formula (V), or a salt or solvate thereof

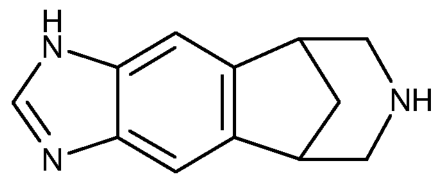

(V)

**59.** Varenicline, or a salt or solvate thereof, having between 0.10% and 0.15% as measured by HPLC of a compound of formula (V), or a salt or solvate thereof

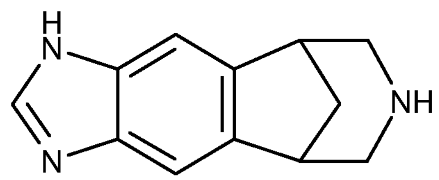

(V)

**60.** Varenicline, or a salt or solvate thereof, according to any of claims 56 to 59, further having the following measurements in the CIE (1976) L*, a*, b* Color Space (CIELAB) when using a colorimeter or spectrophotometer, illuminant D65 and a 2° angle of observation:

d) L* color coordinate value between 2 and 100
e) a* color coordinate value between -3.00 and +3.00; and
f) b* color coordinate value between -20.00 and +20.00.

**61.** Varenicline, or a salt or solvate thereof, according to any of claims 56 to 60, further having a Whiteness Index higher than 50.00, preferably higher than 75.00, preferably higher than 90.00, when using a colorimeter or spectrophotometer, illuminant D65 and a 2° angle of observation.

**62.** A pharmaceutical composition comprising varenicline according to any of claims 56 to 61, together with a pharmaceutically acceptable excipient.

**63.** A method of treating smoking cessation, which comprises administration to a subject varenicline according to any of claims 56 to 61.

**64.** A process for producing a validated batch of varenicline according to any of claims 56 to 61, or a pharmaceutical composition comprising thereof according to claim 62, said process comprising:

a) producing a batch of varenicline, or a pharmaceutical composition comprising thereof;
b) determining the total amount of compound of formula (V), or a salt or solvate thereof,

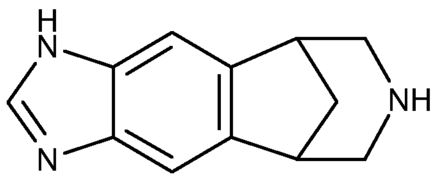

(V)

in a sample of the batch; and
c) validating the batch for distribution only if the sample of the batch contains a suitable concentration of a compound of formula (V), or a salt or solvate thereof, preferably a concentration of less than 0.15% as measured by HPLC, with respect to varenicline.

**65.** A process according to claim 64, wherein the determining the total amount of compound of formula (V), or a salt or solvate thereof, of step b) is carried out through the method according to any of claims 50 to 53.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6410550 B
- US 7091372 B
- US 7186870 B
- WO 2009111623 A
- WO 2006090236 A
- WO 2009155403 A
- WO 2004108725 A
- WO 2010005643 A
- US 20070185327 A